(19) Europäisches Patentamt
European Patent Office
Office européen des brevets

(11) **EP 1 077 677 B1**

(12) **EUROPEAN PATENT SPECIFICATION**

(45) Date of publication and mention
of the grant of the patent:
**22.10.2003 Bulletin 2003/43**

(51) Int Cl.[7]: **A61K 9/00**, A61K 9/70

(21) Application number: **99927062.2**

(86) International application number:
**PCT/SE99/00824**

(22) Date of filing: **12.05.1999**

(87) International publication number:
**WO 99/058109 (18.11.1999 Gazette 1999/46)**

(54) **PROCESS FOR THE MANUFACTURE OF A BIOLOGICALLY ACTIVE COMPOSITION**

VERFAHREN FÜR DIE HERSTELLUNG EINER BIOLOGISCH AKTIVEN ZUSAMMENSETZUNG

PROCEDE DE FABRICATION D'UNE COMPOSITION BIOLOGIQUEMENT ACTIVE

(84) Designated Contracting States:
**AT BE CH CY DE DK ES FI FR GB GR IE IT LI LU MC NL PT SE**

(30) Priority: **14.05.1998 SE 9801705**

(43) Date of publication of application:
**28.02.2001 Bulletin 2001/09**

(73) Proprietor: **JAGOTEC AG**
**4132 Muttenz (CH)**

(72) Inventors:
• **LINDAHL, Åke**
**S-274 33 Skurup (SE)**
• **HAGSLÄTT, Håkan**
**S-224 72 Lund (SE)**
• **HEIMAN, Catharina**
**S-217 56 Malmö (SE)**
• **BRYLAND, Rickard**
**S-217 41 Malmö (SE)**

(74) Representative: **Larsson, Kjell et al**
**AWAPATENT AB,**
**Box 45086**
**104 30 Stockholm (SE)**

(56) References cited:
WO-A1-97/00670          WO-A2-97/10812
GB-A- 2 306 885          US-A- 4 940 701

EP 1 077 677 B1

**Description**

The present invention relates to a process for the manufacture of a biologically active composition from which one or more biologically active components are co be released. More specifically, the invention relates to a biologically active composition wherein the biologically active agent is present in a supersaturated state within a carrier without being precipitated therefrom.

Background of the Invention

From *inter alia* toxicological points of view, it is often preferred, upon treatment of diseases or symptoms thereof, to deliver drugs directly to their site(s) of action. It is well known that the risks of obtaining detrimental effects of systemic origin are often markedly reduced if a drug is delivered directly to its site(s) of action. Furthermore, systemic delivery often involves metabolism of the drug prior to its appearance at the site of action, which leads to a subsequent reduction of its biological effect. Another important aspect is that in *e.g.* cases of imminent overdosage, allergic reactions or administration of contraindicating drugs, it is easy to remove topical compositions in contrast to drugs administered per-orally or by injection.

As used herein, topical administration comprises *inter alia* dermal, sub-lingual, gingival, buccal, transdermal, nasal, vaginal and rectal administration, whereby the resulting biological effect may be local and/or systemic.

In *e.g.* dermal, nasal, vaginal, buccal or sub-lingual administration, only a very limited number of drugs are capable of permeating into the human body by themselves at a useful rate. Consequently, a lot of research has been conducted in order to investigate the possibility of both improving traditional non-invasive delivery techniques and developing novel non-invasive drug delivery systems or devices intended for systemic and/or internal use. Three fundamentally different approaches towards this objective have been disclosed.

Firstly, there is the well known possibility of improving the penetration properties of the drug by chemical modification thereof. After the drug has entered the body, its pharmacologically active form is obtained by chemical reaction(s) *in vivo*. However, this so called pro-drug approach is only occasionally a succesful alternative. There are several reasons therefor, such as *i)* the penetration rate of the pro-drug may still be too low, *ii)* the pro-drug may be toxic or otherwise harmful, or *iii)* the *in vivo* conversion to the active form of the drug is too slow and/or partially results in inactive or toxic compounds. A distantly related approach is the preparation of an ion pair between a drug and an appropriate counter ion. However, generally such an ion pair does not display any markedly improved penetration rate through human barriers.

Secondly, the properties of the barrier may be changed in order to facilitate the drug delivery. Methods of achieving this are *e.g.* ultra-sonication, applying of electrical current or the use of so called penetration enhancers in the composition. All of these methods act by disrupting the structure of the barrier, thereby facilitating drug diffusion through the barrier into the body, and/or improving the drug solubility in the barrier. However, the methods involving *e. g.* heat, ultra-sonication and electrical current are generally not designed for being easily managed by the patient in a convenient manner, and therefore require hospitalisation, which is a major disadvantage with said methods. In addition, all methods which are based on the approach of changing the barrier properties are questionable from a toxicological point of view due to the observations that *i)* adverse effects on the cells of the barrier have been demonstrated, and *ii)* a reduction of the protective properties of the barrier also result in increased penetration rate for any substance, not only the drug, that is present at the site of administration. It should also be mentioned, that a majority of the known chemical penetration enhancers require some time for the onset of their action, *i.e.* display a lag time of action, since they must be established in the barrier before the actual increase in penetration rate is observed.

Thirdly, the driving force of the drug for entering the body can be changed. That is, the difference in the electrochemical potential of the drug between the drug reservoir and the body can be increased. Drug delivery systems based on this approach result in a high flux of the drug through the barrier and usually also display a reduced lag time of action.

In methods based on iontophoresis, this approach is utilised by applying an electrical potential gradient across the barrier. Obviously, these methods are mainly suitable for drugs having a net charge and are therefore much less efficient for uncharged and zwitterionic species, since the flux of the two latter species is improved mainly due to *e.g.* osmotic and electroosmotic driving forces. Iontophoresis methods also have the disadvantage that they may alter the structure of the barrier.

In another approach, the flux of a drug into the body can be enhanced by increasing the chemical potential of the drug in the carrier therefor. This is normally performed by chemical optimisation of the drug composition by adjusting the degree of saturation of the drug in said carrier. The methods based on this approach offer several advantages as compared to the previously mentioned methods, since the flux of the drug is increased in comparison with subsaturated and saturated systems. Furthermore, the properties of the barrier itself are comparatively less affected and the lag time of initiation for the pharmacological effect is reduced. There are two particularly important aspects in this approach:

> i) creation of an initial high chemical potential of the drug in the composition
>
> ii) maintenance of a high chemical potential of the drug in the vicinity of the barrier after the application of the composition.

Therefore, it is usually desirable to prepare pharmaceutical compositions which are saturated with respect of the drug. During application, another important aspect of said composition is that the solubility and diffusion properties of the drug in the used vechicle must preclude depletion of the drug in the vicinity of the barrier. Examples of compositions used for this purpose are microemulsions and emulsions.

Another approach towards keeping the composition saturated is the use of an excess amount of drug (non-solubilised) in the carrier, whereby the drug is subsequently dissolved as it replaces the drug which has penetrated through the barrier.

Yet another approach is the use of a supersaturated composition of the drug. Here, the driving force of the drug to penetrate the barrier is higher than in the saturated composition, since the drug in a supersaturated composition has higher chemical potential in comparison with the corresponding saturated composition. For example, such compositions have been prepared according to the following means or principles: *i)* dissolving the drug at temperatures and/or pressures at which the solubility of the drug is higher as compared to those temperatures and/or pressures that are relevant for medication (W.L. Chou and S. Riegelmann, *J. Pharm. Sci.*, Vol.60, No.9, pp.1281-1302, 1971; WO 97/10812), *ii)* using solid dispersions or eutectic mixtures or solid drug particles of low degree of crystallinity or of high energy polymorphs (W.L. Chou and S. Riegelmann, *supra*), *iii)* mixing a saturated drug solution with a non-solvent therefor, thereby performing a merely physical operation, *in situ* or prior to application, with or without the presence of an antinucleating agent (US 4 940 701; US 4 767 751), *iv)* solvent evaporation to the surrounding air (Coldman *et al., J. Pharm. Sci.*, 58, No.9 (1969), pp 1098-1102), *v)* solvent penetration into the human body, *vi)* water uptake into the composition from the human body, *vii)* pH-changes in the composition caused by $H^+$-uptake from the human body, or *viii)* dispersing an aqueous solution or emulsion of a drug in an aqueous dispersion of a polymer latex (Lichtenberger *et al., "Polymer films from aqueous polymer dispersions as carriers for transdermal delivery of lipophilic drugs",* 15th Int Symp CRS:Basel 1988; Abstr 89). An important common denominator of *iv)-vii)* is that the supersaturation is not initally present in the composition, and is therefore *de facto* not accomplished until the composition is applied to a human body. Furthermore, a major problem with all the compositions *i)-viii)* is that the drug generally precipitates in a relatively short time, in which case the saturation degree becomes markedly reduced.

In DD 217 989, a subsaturated solution of a drug is mixed with a solution or suspension of an acrylate, after with the mixture so prepared is dried, whereby a supersaturated composition is obtained by use of an exclusively physical operation.

W.L. Chou and S. Riegelmann (*J. Pharm. Sci.,* Vol.58, No. 12, pp.1505-1510, 1969) have reported that in matrices of higher molecular weight polyethylene glycols, precipitation of a supersaturated drug dissolved therein is usually sluggish. In said document, supersaturation was obtained through either direct melting or solvent concentration, *i.e.* by use of typical physical operations.

As prior art, reference is also made to WO 97/00670, which discloses a composition based on ingredients similar to those utilized in the present invention. However, said reference does not disclose or suggest any supersaturated state or even less those features and measures of the present invention which have been found crucial to impart a stable, supersaturated state to such a composition.

Other prior art of interest is WO 97/10812, which discloses a method for preparing supersaturated systems, wherein an admixture of drug and polymer having a calculated depressed melting temperature is heated to a temperature above said calculated temperature, whereby the drug is dissolved in the polymeric material and supersaturation thereof is obtained through cooling of the heated solution. However, the present invention is not related to preparation of supersaturated systems by exploitation of the calculated depressed melting temperature of an admixture through an entirely physical operation.

Mention can also be made of GB 2 306 885, which utilises the skin's innate ability to buffer applied liquids. Here, a supersaturated system is attained *in situ* by applying a subsaturated drug composition having a pH of 7-12 or 3-4 to skin, where the buffering effect of skin causes a pH change to 4.5-6.5, whereby a supersaturated composition is obtained by means of a change of the degree of protonation of the drug. The preparation of supersaturated systems according to the present invention does not rely on such an exchange of protons.

General Disclosure of the Invention

A novel process for obtaining a biologically active composition with outstanding delivery rate of its active component(s) has now been developed, wherein said composition comprises a biologically active agent which is present in a substantially stable supersaturated state. In brief summary, it has been found that by subjecting a carrier starting substance to such chemical operation(s) that a carrier matrix of substantially non-crystalline or amorphous nature is created, in which the degree of saturation of a biologically active agent is higher than the degree of saturation of said agent in the starting carrier substance, a surprisingly stable supersaturated composition can be obtained. In the composition thus prepared, the precipitation of said agent is substantially, or completely, inhibited by said carrier matrix *per se.*

The term "biologically active agent", as used herein, also comprises such progenitors thereto which are readily transformable, *e.g.* enzymatically and/or hydrolytically, to a biologically active agent *per se.*

Thus, the present invention relates to a novel process for obtaining a biologically active composition which comprises a biologically active agent to be released therefrom, said biologically active agent being dissolved and/or dispersed in a supersaturated state within a carrier, which carrier is a liquid and/or solid substantially non-crystalline matrix, and where the precipitation of said biologically active agent is substantially, or completely, inhibited therein.

The term "liquid" as used in connection with the present invention should be interpreted in a broad sense, viz as any material being a mobile or viscous liquid, rubber, glass or plastic; thus including solutions, creams, pastes, ointments and gels within the scope of the claims.

The term "pharmaceutically active agent", as used herein, also comprises such progenitors, *e.g.* pro-drugs, which are readily transformable, *e.g.* enzymatically and/or hydrolytically, to a pharmaceutically active agent *per se.*

One of the objects of the present invention is thus to provide a supersaturated composition which does not display any significant precipitation or loss of effect during long-term storage at room temperature, or even at above or below room temperature, during *e.g.* months or even years.

Another object of the present invention is to provide a supersaturated composition which does not display any significant precipitation or loss of effect during its application to a human or animal patient.

Still another object of the present invention is to provide a carrier matrix which is suitable in preparation of a composition having a particularly high degree of supersaturation of a drug (*vide infra*).

Yet another object is to provide a stable supersaturated composition which is easily handled and does not require professional assistance upon use thereof.

As a result of the high delivery rate of its active component(s), another object of the present invention is to provide a composition which allows for efficient topical treatment, preferably dermal or transdermal administration to small areas, which is a general advantage in the topical administration of drugs.

Detailed Disclosure of the Invention

More specifically, the invention refers to a process for obtaining a biologically active composition comprising a biologically active agent dissolved and/or dispersed in a carrier therefor, wherein said carrier is a liquid and/or solid substantially non-crystalline matrix in which said biologically active agent is present in a supersaturated state and in which the precipitation of said biologically active agent is substantially, or completely, inhibited by said matrix, said supersaturated state being obtainable, or obtained, by subjecting one or more starting substance(s) to such chemical operation(s) that a liquid and/or solid substantially non-crystalline matrix is provided in which the degree of saturation of said biologically active agent is increased in comparison with the degree of saturation of said agent in the starting substance(s), the biologically active agent being added before said chemical operation(s) has (have) been completed.

As used herein, the term "chemical operation" refers to a measure resulting in formation or cleavage of covalent bonds. Said formation or cleavage may comprise or by indirect means yield a pH change of the composition, thus involving a proton transfer which in some cases may be regarded as formation or cleavage of a covalent bond. However, such a pH change is in this context the result of a chemical operation which does not merely comprise a proton transfer but which also comprises formation or cleavage of other types of covalent bonds.

In one embodiment of the invention, said supersaturated state is obtainable by subjecting one or more carrier starting substance(s) to such chemical operation(s) that a matrix is provided in which the degree of saturation of said biologically active agent is higher than the degree of saturation of said biologically active agent in said carrier starting substance(s), the biologically active agent being added at a predetermined point of time after said chemical operation (s) have been initiated, after which the composition thus prepared is further subjected to said chemical operation(s).

Other preferable embodiments of the composition claimed will be defined in the claims or referred to below in connection with the method.

Thus, the present invention refers to a method for the preparation of a biologically active composition comprising a biologically active agent dissolved and/or dispersed in a carrier therefor, wherein

a carrier starting substance, or a mixture of two or more different starting substances, is (are) subjected to such chemical operation(s) that a liquid and/or solid non-crystalline carrier matrix is formed, in which the degree of saturation of a biologically active agent is higher than the degree of saturation of said agent in said carrier starting substance(s), said biologically active agent being added before said chemical operation(s) has (have) been completed and in an amount such that a supersaturated state is obtained. Generally this means that said chemical operation(s) is (are) initiated either:

  i) in the presence of said biologically active agent; or
  ii) in the absence of said biologically active agent, after which said agent at a predetermined point of time is added and the composition thus prepared is further subjected to said chemical operation(s);

addition of said biologically active agent in both *i)* and *ii)* being made using an amount such that a supersaturated state is obtained.

In one embodiment of the invention, the degree of saturation of a biologically active agent is higher as a result of such chemical operation(s) that a liquid and/or solid non-crystalline carrier matrix is formed, in which the solubility of a biologically active agent is lower than the solubility of said agent in said carrier starting substance(s).

In another embodiment of the invention, the degree of saturation of a biologically active agent is higher as a result of such chemical operation(s) that a liquid and/or solid non-crystalline carrier matrix is formed, in which the degree of dissociation, aggregation and/or degree of protonation of a biologically active agent is different from the degree of dissociation, aggregation and/or degree of protonation of said agent in said carrier starting substance(s). As a non-limiting example, this embodiment allows formation *in situ* of a suitably charged, *e.g.* protonated or deprotonated, or non-charged form of said biologically active agent, which form has a higher skin penetration rate in comparison with the form of said agent present before said chemical operation(s) is initiated.

In yet another embodiment of the invention, the degree of saturation of a biologically active agent is increased by such chemical operation(s) that both the two embodiments set forth above are practised either simultaneously or consecutively.

In one embodiment of the invention, said biologically active agent is being added, either above or around room temperature, in solid and/or liquid, *i.e.* melted, state and is subsequently dissolved in said starting substance(s) either above or around room temperature.

In another embodiment of the invention, said biologically active agent is being added, either above or around room temperature, as a solution or dispersion and is subsequently dissolved in said starting substance(s) either above or around room temperature.

According to the present invention, above room temperature is a temperature above about 25°C, such as about 25-200°C, preferably about 30-150°C. Examples of other suitable temperatures are about 35-100°C and 40-80°C.

The particular addition method used for said agent can be any common inclusion technique available to a person skilled in the art, and said solution or dispersion of the biologically active agent can be prepared *inter alia* by solvent evaporation, freeze-drying or by use of any one of the methods *i)-vii)* (*vide supra*).

Preferably, in the composition according to the invention as well as in the method for preparation thereof, the starting substance(s) act(s) as solvent or dispersing medium.

Said chemical operation(s) generally involve(s) one or more chemical reactions, preferably etherifying, esterifying, hydrolysis, substitution, addition, elimination, oligomerising and/or polymerising reactions, wherein polymerising reactions are the most preferred.

Said carrier starting substance(s), which is subsequently subjected to said operation(s) above, is selected from monomers, acids, such as mono-, di- or triacids or higher acids, alcohols, including mono-, di-or triols, ketones, aldehydes, amines, amides, anhydrides, lactides, glycolides, saccharides and derivatives thereof, acrylic or acrylamide type compounds, such as methyl methacrylate, monomers of PEO-diacrylate (PEO=polyethylene oxide), cyanoacrylate, acrylate saccharides, including acrylate starch, acrylate lactate, acrylate glycolate, isocyanates, ethylene oxide, propylene oxide, pyrrolidone, PEO-diacrylate, ethylene-vinyl acetate, monomers of organic siloxanes and oligomers, polymers or prepolymers thereof. As indicated earlier, one, two or more of the above substances can be chosen, thereby allowing the formation of co-polymers and/or higher polymers.

It is to be understood by a person skilled in the art, that said chemical operation(s) is performed to such a degree of completion that a desired non-crystalline carrier matrix is obtained, which matrix is optimal for a particular biologically active agent in a particular context. Thus, all of the starting substance(s) present when said chemical operation(s) is initiated do not necessarily have to react completely in order to carry out the invention, as long as the desired degree of supersaturation is attained.

In a preferred embodiment of the present invention, the carrier starting substances are an acid and an alcohol, said formed non-crystalline matrix comprising, or being, an ester and/or polyester thereof. In a more preferred embodiment, said carrier starting substances are citric acid and propylene glycol.

In an alternative embodiment, the starting substance is one bi- or multi-functional substance only, which when subjected to said chemical operation(s) provides the desired non-crystalline carrier matrix by chemical reaction(s) with itself. In a non-limiting disclosure, such a starting substance can be citric acid, which when subjected to esterifying conditions provides a non-crystalline citric acid ester and/or polyester matrix according to the invention.

According to the present invention, suitable chemical operation(s) involve(s) subjecting said carrier starting substance(s) to such polymerising conditions which are normally used, according to standard reference literature, for the selected starting substance(s) or combinations thereof. Furthermore, such polymerising conditions should be chosen in order to optimise the manufacturing procedure, in respect of *e.g.* the scability of said agent, manufacturing time and degree of supersaturation, for the particular biologically active agent used. Typically, said conditions comprise *e.g.* subjecting said carrier starting substance(s) to a temperature from around -50°C to around 300°C, preferably around 0-150°C. Other examples of useful temperature ranges are 20-100°C and 50-80°C. Said temperature ranges are particularly preferred when the starting substance(s) are a mixture of citric acid and propylene glycol. Naturally, said chemical reaction(s) are selected and performed so that in each case the maximum or optimum delivery rate of said biologically active agent is obtained.

Preferably, said chemical reaction(s) is (are) performed for a time period of from 1 minute to 6 months, more preferably from 0,5 hours to 4 months. As an example, said time period may also be from 1 hour to 3 months or from 1 to 2 months.

The predetermined point of time (*vide supra*), as measured after said chemical operation(s) has (have) been initiated, is generally from 1 minute to 6 months, preferably from 0,5 hours to 4 months, after which the composition thus obtained is further subjected to said chemical operation(s) for a time period of about from 1 minute to 6 months, preferably from 0,5 hours to 4 months. As an example, said predetermined point of time may also be from 1 hour to 3 months or from 1 to 2 months.

The used chemical reaction(s) in the present invention preferably comprise a polymerisation reaction and most preferably such reaction in which ether and/or ester bonds are formed. Other preferred polymerisation reactions are step polymerisation reactions and chain polymerisation reactions comprising either radical initiation, ionic initiation or coordination complex initiation.

According to the present invention, some of the monofunctional starting substance(s) above, *e.g.* monoacids and -alcohols, can also be used to form a non-crystalline matrix consisting of *e.g.* monoesters and monoethers. Monofunctional monomers can also be introduced into said chemical reaction as a means of modifying the reaction or controlling the end point thereof.

As already indicated, in order to efficiently inhibit precipitation of the supersaturated biologically active agent, said formed matrix is of a substantially non-crystalline, or amorphous, nature. Polymers, co-polymers, oligomers and ethers or esters of the previosly outlined starting substance(s) (*vide supra*) are particularly useful for this purpose.

A number of different parameters have been of interest in the development of the present invention. As an example of such a parameter, a reaction which results in the formation of a non-crystalline matrix, consisting of molecules with a larger molecular weight than the starting substance(s), can result in an increase of the thermodynamic potential of the form of the biologically active agent(s) which diffuse(s) through a biological barrier, such as skin. During the progression of such a reaction, a lowered solubility of the biologically active agent in said matrix will in many instances be observed, albeit it must here be emphasized that said lowered solubility may not always be necessary in order to yield an increased thermodynamic potential of the form of the biologically active agent which *de facto* diffuses through the skin. Moreover, the degree of dissociation, aggregation and/or protonation of the biologically active agent, *e.g.* as a result of pH changes, is often relevant in eliciting the desired increased thermodynamic potential of the form(s) of said agent diffusing through the skin.

Examples of biologically active agents, preferably pharmaceutically active agents, which are suitable for use in the present invention are *e.g.*, guanosides, corticosteroids, psychopharmaceutical hormones, oxicams, peptides, proteins as well as agents selected from the group of antibiotics, antivirals, antimicrobials, anticancer agents, antifungals, oestrogens, antiinflammatory agents, neuroleptic agents, melanocyte stimulants and gland stimulants, preferably stimulators of sebaceous and pilo-sebaceous glands, and agents with an effect on mast cell secretion.

In an alternative embodiment of the present invention, the biologically active agent may also react reversibly with said starting substance(s) in such a manner that *e.g.* esters, ethers, co-polymers and/or other conjugates are formed. Thus, this embodiment allows preparation of a non-crystalline matrix containing both said biologically active agent in a substantially stable supersaturated state and conjugate(s) thereof, whereas said conjugate(s) can be present in either a subsaturated, saturated or supersaturated state. Alternatively, said conjugate(s) can be present in a supersaturated state, whereas said biologically active agent is present in either a subsaturated, saturated or supersaturated state. Therefore, in the case where said biologically active agent is a drug, this particular embodiment allows formation *in situ* of a corresponding drug progenitor, which may either function as a pro-drug or as a depot of the supersaturated drug, or a combination of both. As an example of this embodiment, a biologically active agent containing a carboxylic acid or alcoholic functionality may form an ester with said carrier starting substance(s) when a mixture thereof is subjected to esterifying conditions.

In another embodiment of the present invention, the starting substance(s) can be an ester and/or polyester matrix, or an ether and/or polyether matrix, to which a biologically active agent is added, after which the dispersion or solution formed is subjected to a hydrolysis reaction providing a liquid and/or solid non-crystalline carrier matrix in which the degree of saturation of said biologically active agent is higher than the degree of saturation of said biologically active agent in said starting substance(s), a stable supersaturated dispersion or solution thus being obtained. As a non-limiting example of such an embodiment, the starting substance(s) may consist of several esters and/or polyesters, of which one or several is much more readily hydrolysed in comparison with all other substances present, including the biologically active agent.

In yet another embodiment of the invention, a minor amount of said starting substance(s) is subjected to said chemical conditions, preferably a polymerisation, in the presence of a solvent, whereby a supersaturated one- or two-phase matrix is formed, such as a liquid/solid non-crystalline matrix.

However, in the most preferred embodiment, the biologically active composition consists of one liquid or solid phase only.

As earlier indicated, in another embodiment of the present invention the carrier starting substance(s) can be subjected to said chemical reaccion(s), preferably a polymerisation, in advance and without the presence of said biologically active agent. By using this approach, a prefabricated liquid and/or solid non-crystalline matrix is provided, to which matrix a biologically active agent can subsequently be added at a predetermined point of time by use of any suitable inclusion method, such as *e.g.* mixing, heating, freeze-drying and/or solvent evaporation, after which the composition thus prepared is further subjected to said chemical reaction(s), which is (are) either identical or somewhat modified, by *e.g.* use of a lower reaction temperature or further addition of one or more of the previously outlined starting substance(s), in comparison with the chemical reaction(s) performed initially.

For some biologically active agents it is preferred to prepare a supersaturated composition shortly before administration thereof. Indeed, the present composition is useful for such preparations in addition to it being suitable for supersaturated compositions intended for long-term storage and application. As for the choice of a suitable degree of supersaturation of the biologically active agent in the present composition, it is known from the laws of thermodynamics that within a given period of time the danger of precipitation increases with the degree of supersaturation. Still, the present composition is also suitable in such particular preparations where a very high degree of supersaturation is desirable, despite a somewhat increased danger of precipitation.

The disclosed invention may optionally be combined with the methods *i)-vii)* (*vide supra*) in any suitable manner, if deemed necessary in any particular case. As an example, the pH of the composition prepared according to the invention may optionally be subsequently modified by inclusion of a suitable acidic or basic compound, if useful in a particular context.

The following example will illustrate the present invention further.

Brief description of the enclosed diagrams

Diagram 1 shows the amount of permeated metronidazole as a function of time for a subsaturated composition $A_0$, a saturated composition C and the supersaturated compositions $B_1$ and $B_2$.

Diagram 2 displays the amount of metronidazole permeated from compositions X1-X4 and Y1-Y4.

Experimental part

Example 1; demonstrating an increased thermodynamic potential by using the method of the present invention:

The degree of supersaturation was characterised by the permeation race of the biologically active agent through a membrane (Silastic sheeting NRV, 0,005 inches, serial #HH055353) by using a Franz diffusion cell (FDC-400 Crown Glass Company) with a cell opening area of 2,011 $cm^2$. All permeation rate measurements were performed at 25°C and deaerated $H_2O$ was used as acceptor phase on the opposing side of the membrane. The donor and acceptor phase were both sealed with parafilm, and each experiment was performed in triplicate.

Starting substances: citric acid (CiAc) and propylene glycol

Four parts of CiAc and six parts of propylene glycol were added to a sealable container at room temperature, after which said container was sealed. The resulting mixture was stirred with a magnetic stirrer and the temperature was raised to and maintained at 80°C until all CiAc was dissolved, after which the solution was allowed to attain room temperature. This solution was denoted A. Solid metronidazole was then added to the solution A in a 5:95 ratio (w/w), after which the metronidazole was dissolved by magnetic stirring at room temperature. The solution thus prepared was then split into two solutions denoted $A_0$ and B, respectively.

As reference, a solution of 4 parts of CiAc and 6 parts of propylene glycol was prepared as above. Solid metronidazole in a 7,5:92,5 ratio (w/w) was added, and the mixture was stirred at room temperature for three days.

After centrifugation resulting in sedimentation of non-dissolved metronidazole, the obtained supernatant thus consisted of a saturated metronidazole composition, denoted C. The obtained final ratio between metronidazole and CiAc/propylene glycol was 7:93 (w/w).

The underlying principles behind the compositions A-C were the following:

$A_0$ is a *subsaturated* mixture of a pharmaceutically active agent and carrier starting substances which is not actively subjected to polymerisation;

in B, the starting substances are subjected to polymerisation conditions in the presence of a pharmaceutically active agent; and

in C, the permeation rate for a saturated solution of a pharmaceutically active agent in a matrix of said carrier starting substances is illustrated.

The compositions B and C were then treated as follows:

B was split into two compositions, which were stored at 70°C for one month ($B_1$) and two months ($B_2$), respectively, after which time period the permeation rate measurements were performed on the formed compositions $B_1$ and $B_2$, respectively.

The compositions $A_0$ and C were used directly after the preparation thereof.

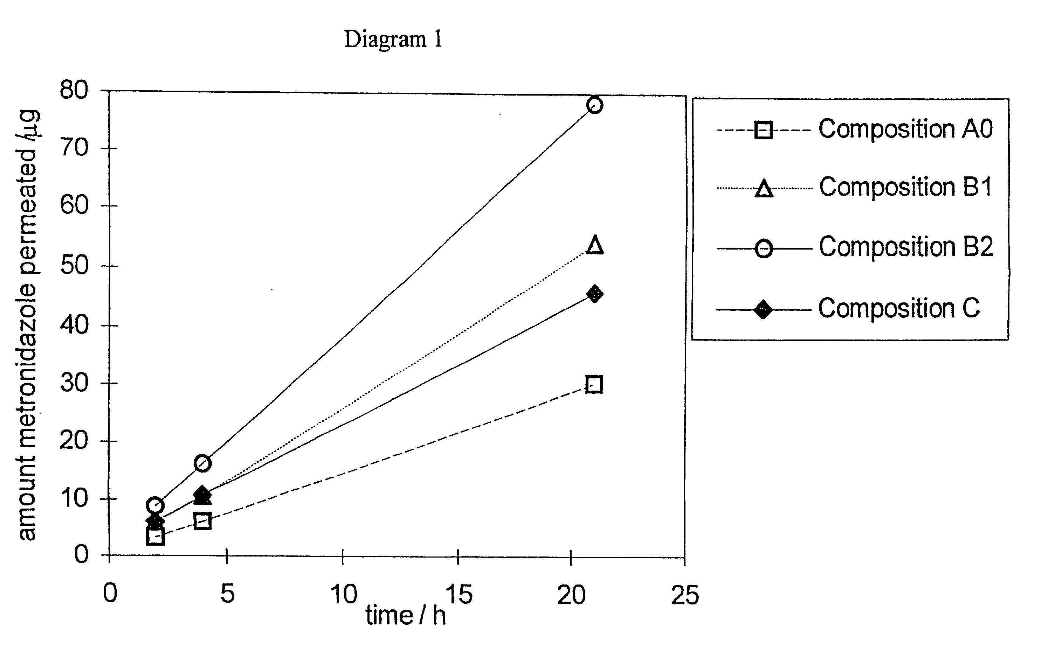

The measured permeation rates are depicted in the enclosed Diagram 1.

Diagram 1 shows that a considerably higher permeation rate is obtained in the compositions $B_1$ and $B_2$, as compared to any one of the compositions A or C. This increased permeation rate is in turn clear evidence that the thermodynamic potential of metronidazole is significantly higher in the compositions $B_1$ and $B_2$ in comparison with any one of the compositions $A_0$ or C. Here, it is important to note that the compositions $A_0$ and B are initially the same.

In summary, this example shows that supersaturation of the initially subsaturated composition is attained upon polymerisation. Indeed, further polymerisation results in an even higher permeation rate, *i.e.* a higher thermodynamic potential, as is illustrated by $B_1$ and $B_2$.

Example 2; demonstrating the precipitation preventing properties of the carrier matrix of the present invention:

A Franz diffusion cell as disclosed above was used under conditions similar to those of example 1, unless otherwise noted. Permeation rate experiments were performed for 21 h. As a reference, the permeation rate from the saturated composition C in example 1 was determined to be 46 µg per 21 h in a Franz diffusion cell experiment, as depicted in Diagram 1. The experiments were all analysed by use of spectrophotometry. The results are depicted in diagram 2.

In order to determine its water solubility, an excess of metronidazole was added to water, after which the mixture was stirred for 3 days at room temperature. Analysis by spectrophotometry was performed after sedimentation and centrifugation, and a resulting solubility of s=0.82% (w/w) was obtained.

Four supersaturated water solutions of metronidazole were then manufactured, each one having a degree of saturation (DS = concentration/solubility) of 1.3, 1.6, 2.0 and 2.5, respectively. They were prepared by heating the corresponding amount of metronidazole in water to 80°C for 30 min under stirring, followed by equilibration to room temperature, thereby yielding supersaturated solutions. The time for precipitation of metronidzole to occur ($t_p$) upon storage at room temperature was monitored by visual inspection, and the results are shown in Table 1.

Table 1.

| Time for precipitation of metronidazole from a supersaturated solution thereof in water. | | | |
|---|---|---|---|
| Solution | metronidazole conc. % (w/w) | DS* | $t_p$ |
| 1 | 1.06 | 1.3 | 5 days < $t_p$ < 14 days |
| 2 | 1.31 | 1.6 | 2 h < $t_p$ < 17 h |
| 3 | 1.65 | 2.0 | 3 h < $t_p$ < 3.5 h |
| 4 | 2.05 | 2.5 | 0.5 h < $t_p$ < 1 h |

*DS=1 equals 0.82 % (w/w) metronidazole in water *(vide supra)*, as determined by spectrophotometry

A composition X was manufactured by mixing 4 parts of CiAc and 6 parts of propylene glycol (starting substances) at room temperature in a glass container which was subsequently sealed. The temperature was raised to and maintained at 80°C under stirring for about 45 min. The resulting solution was kept at room temperature for about 30 min, and was then split into 4 separate solutions. An appropriate amount of metronidazole (see Table 2) was subsequently added to each solution, followed by heating of the mixture at 80°C for about 40 min, after which the resulting compositions were allowed to attain room temperature, thereby yielding the supersaturated compositions X1-X4. Directly after their preparation, the compositions X1-X4 were investigated by Franz diffusion cell measurements (see example 3).

A sample was taken from each respective composition X1-X4. These four samples were each kept at 70°C for 3 weeks, thereby yielding the compositions Y1-Y4 (see Table 2). Y1-Y4 were also examined in Franz diffusion cell experiments (see example 3).

Table 2.

| Degree of saturation of metronidazole in the compositions X1-X4 and Y1-Y4. | | | | |
|---|---|---|---|---|
| metronidazole conc. % (w/w) | Composition | DS* | Composition | DS* |
| 8.0 | X1 | 1.16 | Y1 | 1.62 |
| 9.0 | X2 | 1.23 | Y2 | 1.86 |
| 10.0 | X3 | 1.54 | Y3 | 2.02 |
| 11.0 | X4 | 1.59 | Y4 | 2.18 |

*The permeation rate at saturation was assumed to be 46 μg per 21 h.

The DS values shown in Table 2 were obtained by use of Franz diffusion cell measurements, and to a person skilled in the art, it is well known that the permeation rate of a compound through a Silastic membrane in a Franz cell diffusion cell experiment is a direct measure of the thermodynamic potential of said compound. Moreover, a direct correlation between the thermodynamic potential and the degree of saturation (DS) can often be assumed. Therefore, the equation

$$DS = \text{permeation rate/permeation rate at saturation}$$

was therefore assumed to be valid when estimating the DS

values.

The $t_p$-values for the compositions Y1-Y4 according to the present invention were then investigated in the same manner as described above. These investigations showed that the $t_p$ value for all the compositions Y1-Y4 exceeds 6 weeks. At the time of filing the present application, no precipitation had yet been observed. Indeed, the precipitation preventing properties of the carrier matrix according to the present invention were clearly substantiated, particularly in comparison with the $t_p$ values depicted in Table 1 above.

Example 3; further evidence of increased thermodynamic potential attained in accordance with the present invention:

These experiments were performed in order to further substantiate the degree of saturation of metronidazole in the compositions X1-X4 and Y1-Y4. The Franz diffusion cell experiments were performed under the same conditions as in example 1 (*vide supra*), and the results are shown in Diagram 2.

Diagram 2. Amount of metronidazole permeated from compositions X1-X4 and Y1-Y4.

As depicted above, Diagram 2 shows that the chemical operation subjected to the compositions X1-X4 upon manufacturing of the compositions Y1-Y4 resulted in an increased thermodynamic potential of metronidazole, as is directly evidenced through the increased permeation rate. The permeation rate for a Y composition has increased approximately 40% in comparison with its corresponding X composition.

In summary, it is clearly realised that biologically active compositions which are prepared by the process of the present invention are useful as medicaments. Furthermore, the biologically active compositions prepared by the process of the invention are also useful in a non-medicinal context, such as in cosmetic skin products. More specifically, said compositions should be highly efficient in dermal application to a mammal, preferably man, as well as in any general application where a biological barrier is to be penetrated by a biologically active agent.

## Claims

1. Process for the manufacture of a biologically active composition comprising a biologically active agent to be released therefrom, said biologically active agent being dissolved and/or dispersed in a carrier therefor, wherein said carrier is a liquid and/or solid non-crystalline matrix in which said biologically active agent is present in a supersaturaced state, said supersaturated state being obtained by subjecting one or more carrier starting substance(s) to such chemical operation(s) that said liquid and/or solid non-crystalline carrier matrix is provided in which the degree of saturation of said biologically active agent is higher than in said carrier starting substance(s), the biologically active agent being added before said chemical operation(s) has (have) been completed, the chemical operation resulting in the formation or cleavage of covalent bonds.

2. A process according to claim 1, wherein said higher degree of saturation is the result of such chemical operation(s) that the solubility of the biologically active agent in said matrix is lower than the solubility thereof in said carrier starting substance(s).

3. A process according to any one of claims 1 and 2, wherein said higher degree of saturation is the result of such chemical operation(s) that the degree of dissociation, aggregation and/or degree of protonation of the biologically active agent is different from the degree of dissociation, aggregation and/or degree of protonation of said agent in said carrier starting substance(s).

4. A process according to any one of claims 1-3, wherein said biologically active agent is added before said chemical operation(s) has (have) been initiated.

5. A process according to any one of claims 1-3, wherein said biologically active agent is added at a predetermined point of time after said chemical operation(s) has (have) been initiated, the composition thus obtained then being further subjected to said chemical operation(s).

6. A process according to claim 5, wherein said predetermined point of time is from 1 minute to 6 months, preferably from 0,5 hours to 4 months after said chemical operation(s) has (have) been initiated.

7. A process according to claim 6, wherein the composition is further subjected to said chemical operation(s) for a time period of about from 1 minute to 6 months, preferably from 0,5 hours to 4 months.

8. A process according to any one of claims 1-7, wherein said starting substance(s), or said formed non-crystalline matrix, act(s) as a solvent or dispersing medium.

9. A process according to any one of claims 1-8, wherein said biologically active agent is added as a solid and/or liquid which is subsequently dissolved in said carrier.

10. A process according to any one of claims 1-8, wherein said biologically active agent is added in the form of a solution or dispersion.

11. A process according to any one of claims 1-10, wherein said biologically active agent is added above or around room temperature.

12. A process according to any one of claims 1-11, wherein said chemical operation(s) comprise one or more chemical reactions.

13. A process according to claim 12, wherein said chemical reaction(s) comprise etherifying, esterifying, hydrolysis, substitution, addition, elimination, oligomerising and/or polymerising reactions.

14. A process according to claim 13, wherein said chemical reaction(s) is (are) selected and performed so as to provide optimal delivery rate of said biologically active agent.

15. A process according to any one of claims 1-14, wherein said chemical operation(s) involve(s) subjecting said carrier starting substance(s) to a temperature of from around -50°C to around 300°C, preferably around 0-150°C.

16. A process according to any one of claims 1-15, wherein said chemical operation(s) is (are) conducted for a time period of from 1 minute to 6 months, preferably from 0,5 hours to 4 months.

17. A process according to any one of claims 1-16, wherein said carrier starting substance, or mixture of two or more different carrier starting substances, is selected from monomers, acids, such as mono-, di- or triacids or higher acids, alcohols, including mono-, di-or triols, ketones, aldehydes, amines, amides, anhydrides, lactides, glycolides, saccharides and derivatives thereof, acrylic or acrylamide type compounds, such as methyl methacrylate, monomers of PEO-diacrylate, cyanoacrylate, acrylate saccharides, including acrylate starch, acrylate lactate, acrylate glycolate, isocyanates, ethylene oxide, propylene oxide, pyrrolidone, PEO-diacrylate, ethylene-vinyl acetate, monomers of organic siloxanes, and oligomers, polymers or prepolymers thereof.

18. A process according to claim 17, wherein the acid is a monomeric acid and the alcohol is a monomeric alcohol, said non-crystalline matrix comprising an ester and/or polyester thereof.

19. A process according to claim 18, wherein said monomeric acid is citric acid.

20. A process according to any one of claims 18 and 19, wherein said monomeric alcohol is propylene glycol.

21. A process according to any one of the preceding claims, wherein the composition consists of one liquid or solid phase only.

22. A process according to any one of the preceding claims, wherein the biologically active agent is a pharmaceutically active agent.

23. A process according to claim 22, wherein the pharmaceutically active agent is selected from the group consisting of guanosides, corticosteroids, psychopharmaceutical hormones, oxicams, peptides, proteins, antibiotics, antivirals, antimicrobials, anticancer agents, antifungals, oestrogens, antiinflammatory agents, neuroleptic agents, melanocyte stimulants and gland stimulants, preferably stimulators of sebaceous and pilo-sebaceous glands, and agents with an effect on mast cell secretion.

24. A process for the manufacture of a composition for topical, preferably dermal application to a mammal, preferably man, which includes the process according to any one of the preceeding claims.


**Patentansprüche**

1. Verfahren zur Herstellung einer biologisch wirksamen Zusammensetzung, umfassend einen biologischen Wirkstoff, welcher daraus freigesetzt werden soll, wobei der biologische Wirkstoff in einem Träger dafür gelöst und/oder dispergiert ist, wobei der Träger eine flüssige und/oder feste nicht-kristalline Matrix ist, in welcher der biologische Wirkstoff in einem übersättigten Zustand vorhanden ist, wobei der übersättigte Zustand erhalten wird, indem eine oder mehrere Träger-Ausgangssubstanz(en) derartigen chemischen Verfahren unterworfen werden, so dass die flüssige und/oder feste nicht-kristalline Trägermatrix bereitgestellt wird, in welcher der Sättigungsgrad des biologischen Wirkstoffs höher ist als in der bzw. den Träger-Ausgangssubstanz(en), wobei der biologische Wirkstoff vor Beendigung des (der) chemischen Verfahren(s) zugegeben wird, wobei das chemische Verfahren zur Bildung oder Spaltung von kovalenten Bindungen führt.

2. Verfahren gemäß Anspruch 1, wobei der höhere Sättigungsgrad das Ergebnis eines oder mehrerer derartiger chemischer Verfahren ist, so dass die Löslichkeit des biologischen Wirkstoffs in der Matrix geringer ist als die Löslichkeit desselben in der bzw. den Träger-Ausgangssubstanz(en).

3. Verfahren gemäß einem der Ansprüche 1 und 2, wobei der höhere Sättigungsgrad das Ergebnis eines oder mehrerer derartiger chemischer Verfahren ist, so dass sich der Dissoziations-, Aggregations- und/oder Protonierungsgrad des biologischen Wirkstoffs von dem Dissoziations-, Aggregations- und/oder Protonierungsgrad des Wirkstoffs in der bzw. den Träger-Ausgangssubstanz(en) unterscheidet.

4. Verfahren gemäß einem der Ansprüche 1 bis 3, wobei der biologische Wirkstoff zugegeben wird, bevor das bzw. die chemischen Verfahren eingeleitet werden.

5. Verfahren gemäß einem der Ansprüche 1 bis 3, wobei der biologische Wirkstoff zu einem vorher festgelegten Zeitpunkt nach Einleiten des bzw. der chemischen Verfahren zugegeben wird, wobei die so erhaltene Zusammensetzung dann weiter dem bzw. den chemischen Verfahren unterworfen wird.

6. Verfahren gemäß Anspruch 5, wobei der vorher festgelegte Zeitpunkt bei 1 Minute bis 6 Monaten, vorzugsweise bei 0,5 Stunden bis 4 Monaten nach Einleiten des bzw. der chemischen Verfahren liegt.

7. Verfahren gemäß Anspruch 6, wobei die Zusammensetzung weiterhin dem bzw. den chemischen Verfahren über einen Zeitraum von etwa 1 Minute bis 6 Monaten, vorzugsweise von 0,5 Stunden bis 4 Monaten, unterworfen wird.

8. Verfahren gemäß einem der Ansprüche 1 bis 7, wobei die Ausgangssubstanz(en) oder die gebildete nicht-kristalline Matrix als Lösungsmittel oder Dispergiermittel dient bzw. dienen.

9. Verfahren gemäß einem der Ansprüche 1 bis 8, wobei der biologische Wirkstoff als Feststoff und/oder Flüssigkeit zugegeben wird, welcher anschließend in dem Träger gelöst wird.

10. Verfahren gemäß einem der Ansprüche 1 bis 8, wobei der biologische Wirkstoff in Form einer Lösung oder Dispersion zugegeben wird.

11. Verfahren gemäß einem der Ansprüche 1 bis 10, wobei der biologische Wirkstoff oberhalb oder bei etwa Raumtemperatur zugegeben wird.

12. Verfahren gemäß einem der Ansprüche 1 bis 11, wobei das bzw. die chemischen Verfahren eine oder mehrere chemische Reaktionen umfassen.

13. Verfahren gemäß Anspruch 12, wobei die chemische(n) Reaktion(en) Veretherungs-, Veresterungs-, Hydrolyse-, Substitutions-, Additions-, Eliminierungs-, Oligomerisierungs- und/oder Polymerisierungsreaktionen umfassen.

14. Verfahren gemäß Anspruch 13, wobei die chemische(n) Reaktion(en) so gewählt und durchgeführt wird bzw. werden, dass eine optimale Zufuhrgeschwindigkeit des biologischen Wirkstoffs bereitgestellt wird.

15. Verfahren gemäß einem der Ansprüche 1 bis 14, wobei das bzw. die chemische(n) Verfahren beinhaltet bzw. beinhalten, dass die Träger-Ausgangssubstanz(en) einer Temperatur von etwa -50°C bis etwa 300°C, vorzugsweise etwa 0 bis 150°C, ausgesetzt wird bzw. werden.

16. Verfahren gemäß einem der Ansprüche 1 bis 15, wobei die chemische(n) Reaktion(en) über einen Zeitraum von 1 Minute bis 6 Monaten, vorzugsweise von 0,5 Stunden bis 4 Monaten, durchgeführt wird bzw. werden.

17. Verfahren gemäß einem der Ansprüche 1 bis 16, wobei die Träger-Ausgangssubstanz oder ein Gemisch von zwei oder mehreren verschiedenen Träger-Ausgangssubstanzen aus Monomeren, Säuren, wie z.B. Mono-, Di- oder Trisäuren oder höheren Säuren, Alkoholen, einschließlich Mono-, Di- oder Triolen, Ketonen, Aldehyden, Aminen, Amiden, Anhydriden, Lactiden, Glycoliden, Sacchariden und Derivaten davon, Acrylsäure- oder Acrylamid-Verbindungen, wie z.B. Methylmethacrylat, Monomeren aus PEO-Diacrylat, Cyanoacrylat, Acrylatsacchariden, einschließlich Acrylatstärke, Acrylatlactat, Acrylatglycolat, Isocyanaten, Ethylenoxid, Propylenoxid, Pyrrolidon, PEO-Diacrylat, Ethylen-Vinylacetat, Monomeren aus organischen Siloxanen und Oligomeren, Polymeren oder Präpolymeren davon ausgewählt ist.

18. Verfahren gemäß Anspruch 17, wobei die Säure eine monomere Säure ist und der Alkohol ein monomerer Alkohol ist, wobei die nicht-kristalline Matrix einen Ester und/oder Polyester davon umfasst.

19. Verfahren gemäß Anspruch 18, wobei die monomere Säure Citronensäure ist.

20. Verfahren gemäß einem der Ansprüche 18 und 19, wobei der monomere Alkohol Propylenglycol ist.

21. Verfahren gemäß einem der vorstehenden Ansprüche, wobei die Zusammensetzung aus nur einer flüssigen oder festen Phase besteht.

22. Verfahren gemäß einem der vorstehenden Ansprüche, wobei der biologische Wirkstoff ein pharmazeutischer Wirkstoff ist.

23. Verfahren gemäß Anspruch 22, wobei der pharmazeutische Wirkstoff aus Guanosiden, Corticosteroiden, psychopharmazeutischen Hormonen, Oxicamen, Peptiden, Proteinen, Antibiotika, Antivirenmitteln, antimikrobiellen Mitteln, Antikrebsmitteln, Fungiziden, Östrogenen, entzündungshemmenden Mitteln, Neuroleptika, Melanocyt-Stimulantien und Drüsen-Stimulantien, vorzugsweise Stimulatoren für Talg- und Pilotalgdrüsen, und Mitteln mit einer Wirkung auf die Mastzellen-Sekretion ausgewählt ist.

24. Verfahren zur Herstellung einer Zusammensetzung zur topischen, vorzugsweise zur dermalen Anwendung bei einem Säugetier, vorzugsweise einem Menschen, welches das Verfahren gemäß einem der vorstehenden Ansprüche einschließt.

## Revendications

1. Procédé de fabrication d'une composition biologiquement active comprenant un agent biologiquement actif destiné à en être libéré, ledit agent biologiquement actif étant dissous et/ou dispersé dans un support pour celui-ci, où ledit support est une matrice non cristalline liquide et/ou solide, dans laquelle ledit agent biologiquement actif est présent dans un état sursaturé, ledit état sursaturé étant obtenu en soumettant une ou plusieurs substances initiales de support à une ou plusieurs opérations chimiques telles qu'il se forme ladite matrice de support non cristalline liquide et/ou solide dans laquelle le degré de saturation dudit agent biologiquement actif est plus élevé que dans ladite ou lesdites substances initiales de support, ledit agent biologiquement actif étant ajouté avant que ladite ou lesdites opérations chimiques aient été achevées, l'opération chimique conduisant à la formation due au clivage de liaisons covalentes.

**2.** Procédé selon la revendication 1, où ledit plus haut degré de saturation est le résultat d'une ou plusieurs opérations chimiques telles que la solubilité de l'agent biologiquement actif dans ladite matrice est inférieure à sa solubilité dans ladite ou lesdites substances initiales de support.

**3.** Procédé selon l'une quelconque des revendications 1 et 2, où ledit plus haut degré de saturation est le résultat d'une ou plusieurs opérations chimiques telles que le degré de dissociation, d'agrégation et/ou le degré de protonation de l'agent biologiquement actif est différent du degré de dissociation, d'agrégation et/ou du degré de protonation dudit agent dans ladite ou lesdites substances initiales de support.

**4.** Procédé selon l'une quelconque des revendications 1 à 3, où ledit agent biologiquement actif est ajouté avant que ladite ou lesdites opérations chimiques aient été commencées.

**5.** Procédé selon l'une quelconque des revendications 1 à 3, où ledit agent biologiquement actif est ajouté à un moment prédéterminé après que ladite ou lesdites portions chimiques ont été commencées, la composition ainsi obtenue étant soumise encore à ladite ou auxdites opérations chimiques.

**6.** Procédé selon la revendication 5, où ledit moment prédéterminé est de 1 min à 6 mois, de préférence de 0,5 h à 4 mois après que ladite ou lesdites opérations chimiques ont été commencées.

**7.** Procédé selon la revendication 6, où la composition est soumise encore à ladite ou auxdites opérations chimiques pendant une durée d'environ 1 min à 6 mois, de préférence de 0,5 h à 4 mois.

**8.** Procédé selon l'une quelconque des revendications 1 à 7, où ladite ou lesdites substances initiales, ou ladite matrice non cristalline formée, jouent le rôle de solvant ou de milieu dispersant.

**9.** Procédé selon l'une quelconque des revendications 1 à 8, où ledit agent biologiquement actif est ajouté sous forme d'un solide et/ou d'un liquide qui est ensuite dissous dans ledit support.

**10.** Procédé selon l'une quelconque des revendications 1 à 8, où ledit agent biologiquement actif est ajouté sous forme d'une solution ou dispersion.

**11.** Procédé selon l'une quelconque des revendications 1 à 10, où ledit agent biologiquement actif est ajouté audessus ou au voisinage de la température ambiante.

**12.** Procédé selon l'une quelconque des revendications 1 à 11, où ladite ou lesdites opérations chimiques comprennent une ou plusieurs réactions chimiques.

**13.** Procédé selon la revendication 12, où ladite ou lesdites réactions chimiques comprennent des réactions d'éthérification, d'estérification, d'hydrolyse, de substitution, d'addition, d'élimination, d'oligomérisation et/ou de polymérisation.

**14.** Procédé selon la revendication 13, où ladite ou lesdites réactions chimiques sont choisies et conduites de manière à assurer la vitesse de délivrance optimale dudit agent biologiquement actif.

**15.** Procédé selon l'une quelconque des revendications 1 à 14, où ladite ou lesdites opérations chimiques comprennent la soumission de ladite ou desdites substances initiales de support à une température d'environ -50°C à environ 300°C, de préférence d'environ 0-150°C.

**16.** Procédé selon l'une quelconque des revendications 1 à 15, où ladite ou lesdites opérations chimiques sont conduites pendant une durée de 1 min à 6 mois, de préférence de 0,5 h à 4 mois.

**17.** Procédé selon l'une quelconque des revendications 1 à 16, où ladite substance initiale de support, ou un mélange de deux ou plusieurs substances initiales de support différentes, est choisi parmi les monomères, les acides, comme les mono- di- ou triacides ou les acides supérieurs, les alcools, y compris les mono- di- ou triols, les cétones, les aldéhydes, les aminés, les amides, les anhydrides, les lactides, les glycolides, les saccharides et leurs dérivés, les composés de type acrylique ou acrylamide comme le méthacrylate de méthyle, les monomères de PEO-diacrylate, le cyanoacrylate, les saccharides acrylates, y compris l'amidon acrylate, le lactate acrylate, le glycolate acrylate, les isocyanates, l'oxyde d'éthylène, l'oxyde de propylène, la pyrrolidone, le PEO-diacrylate, l'éthylène-acétate de vinyle, les monomères de siloxanes organiques, et leurs oligomères, polymères ou prépolymères.

18. Procédé selon la revendication 17, où l'acide est un acide monomère et l'alcool est un alcool monomère, ladite matrice non cristalline comprenant un ester et/ou polyester de ceux-ci.

19. Procédé selon la revendication 18, où ledit acide monomère est l'acide citrique.

20. Procédé selon l'une quelconque des revendications 18 et 19, où ledit alcool monomère et le propylèneglycol.

21. Procédé selon l'une quelconque des revendications précédentes où la composition consiste en une phase liquide ou solide seulement.

22. Procédé selon l'une quelconque des revendications précédentes, où l'agent biologiquement actif est un agent pharmaceutiquement actif.

23. Procédé selon la revendication 22, où l'agent pharmaceutiquement actif est choisi dans le groupe consistant en les guanosides, les corticostéroïdes, les hormones psychopharmaceutiques, les oxicames, les peptides, les protéines, les antibiotiques, les antiviraux, les antimicrobiens, les agents anticancéreux, les antifongiques, les oestrogènes, les agents anti-inflammatoires, les agents neuroleptiques, les stimulants des mélanocytes et les stimulants des glandes, de préférence les stimulants des glandes sébacées et pilo-sébacées, et les agents ayant un effet sur la sécrétion des mastocytes.

24. Procédé de fabrication d'une composition pour l'application topique, de préférence dermique, à un mammifère, de préférence l'homme, qui inclut le procédé selon l'une quelconque des revendications précédentes.

Diagram 1

EP 1 077 677 B1